# DEMANDE DE BREVET EUROPEEN

(11) **EP 0 955 051 A1**
(43) Date de publication de la demande: **10.11.1999**
(21) Numéro de dépôt: 99401049.4
(22) Date de dépôt: 29.04.1999
(51) Int. Cl.: A61K 33/34, A61K 33/24, A61K 33/00

(54) **Composition renfermant des oligomères procyanidoliques (OPC) et un cation divalent**

(30) Priorité: 29.04.1998 FR 9805386
(71) Demandeur: Laboratoires Pharmascience, 92400 Courbevoie (FR)
(72) Inventeur: Msika, Philippe, 75018 Paris (FR); Tep Saly, Thida, 94200 Ivry sur Seine (FR)
(74) Mandataire: Texier, Christian

(57) **Abrégé**

La présente invention concerne une nouvelle composition renfermant des oligomères procyanidoliques (OPC) et un cation divalent, notamment un ion cuivre divalent.

Elle couvre également l'utilisation d'une telle composition comme antifongique, par exemple en cosmétique et en dermatologie en particulier pour la prévention ou le traitement de l'érythème fessier et des candidoses, ou en tant que complément alimentaire.

## Description

La présente invention concerne une composition renfermant des oligomères procyanidoliques (OPC) en association avec un cation divalent et l'utilisation d'une telle composition en tant qu'agent antifongique, en tant que produit cosmétique ou pharmaceutique, ou encore en tant que complément alimentaire.

Les oligomères procyanidoliques sont présents dans des extraits végétaux titrés en proanthocyanidol dont l'utilisation dans le domaine médical, diététique ou cosmétique a déjà été largement exploitée.

Leur nature et leur composition sont définies par leur mode d'extraction, qui permet d'isoler des végétaux considérés une fraction présentant l'activité biologique recherchée. Les proanthocyanidols responsables de l'activité sont des polyphénols appartenant à la série du flavane-3 ol. Ils se présentent dans la plante sous divers degrés de polymérisation dont certains seulement sont doués d'une activité intéressante. Les extraits bruts de proanthocyanidols sont formés de mélanges complexes comprenant des monomères et des polymères, plus particulièrement des oligomères allant des dimères et le plus généralement jusqu'aux décamères. Les procédés d'extraction visent à privilégier l'obtention des oligomères c'est-à-dire les formes peu polymérisées, les polymères supérieurs ne présentant généralement comme intérêt que leur pouvoir tannant. En pratique, les oligomères d'intérêt réunissent de deux à quatre molécules mères. L'association des monomères se fait le plus généralement par des liaisons interflavaniques carbone - carbone 4 - 6 ou 4 - 8. Ces polymères ont été isolés ou identifiés dans tous les groupes végétaux, gymnospermes et fougères compris. On les trouve par exemple dans le quebracho, la vigne, le pin, le thé, l'arachide, l'aubépine ou le cyprès. La formule ci-dessous donne un exemple de dimère (procyanidol).

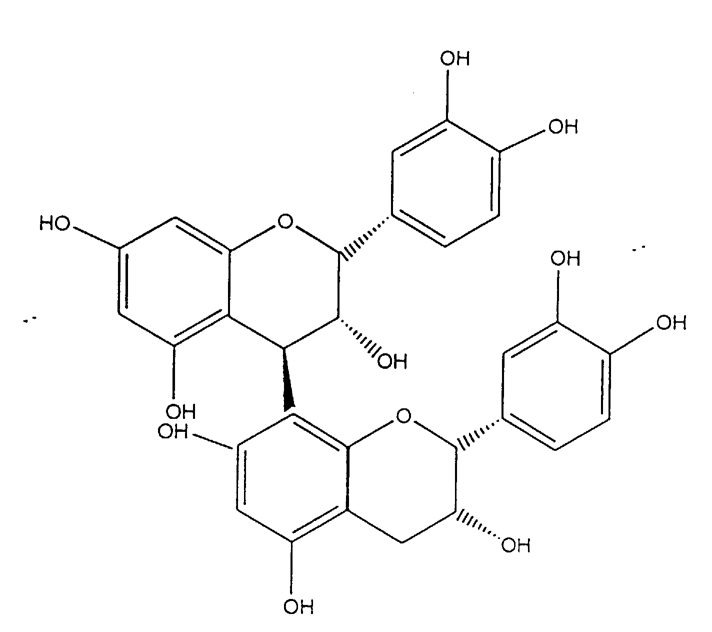

La publication de brevet FR 2 715 582 contient une définition d'OPC utilisables selon l'invention.

On connaît déjà certaines applications antifongiques ou antibactériennes des OPC. Ainsi par exemple, la publication de brevet EP-A-397914 décrit l'utilisation des polyphénols de thé comme antitoxine contre des exotoxines de bactéries telles que celles produites par Clostridium botulinum, Vibrio cholerae, Vibrio parahaemolyticus et Staphylococcus aureus.

La publication de brevet EP-A-443090 décrit également l'action bactéricide du thé ou des polyphénols de thé contre une souche de Staphylococcus aureus résistant à la méthicilline.

Par ailleurs, au cours de travaux datant de 1971, Dumas et Masquelier (Anal. Inst. Pasteur, 1971, 121, 69-73) ont étudié l'activité levuricide sur Candida albicans d'un mélange de leucocyanidol et de chlorure cuivrique.

Le leucocyanidol est un précurseur d'OPC très répandu chez les végétaux. Il s'agit d'un flavane diol-3,4 (flavane-3,4 ol) qui constitue un produit de réduction d'un flavane-3 ol, lequel, comme rappelé ci-dessus, représente l'élément structural de base des OPC. Il est obtenu à partir d'un tégument de l'arachide ou du pin maritime. Lorsque le leucocyanidol est oxydé, il se produit une polymérisation conduisant à la formation d'oligomères. Il est connu que dans certains cas, de tels produits d'oxydation ont une activité antifongique. Ainsi, par exemple, la publication Cole Nature, 1958, 4623, 1696-1697 indique que les polymères, produits de l'oxydation des leuco-anthocyanidols, ont une activité d'inhibition de la polygalacturonase, enzyme produite par Sclérotinia fructigena, responsable du pourissement des pommes.

Dans leurs travaux de 1971, Dumas et Masquelier ont comparé l'activité levuricide sur la levure Candida albicans du leucocyanidol associée au chlorure cuivrique lorsque l'on fait varier le temps pendant lequel le leucocyanidol et le chlorure cuivrique sont restés en contact avant l'introduction de la levure. Lorsque la levure est ajoutée au mélange leucocyanidol-CuCl₂ après un temps de contact très faible entre les constituants du mélange, l'activité levuricide est significative. Par contre, il apparaît que l'activité antifongique diminue considérablement lorsque le temps de contact préalable augmente.

Dumas et Masquelier émettent l'hypothèse que les ions cuivriques oxydent les molécules du flavanol qui réagissent ensuite entre elles en se polymérisant. Selon leurs résultats, les produits d'oxydation, c'est-à-dire les polymères obtenus après un temps de contact prolongé entre le flavanol (leucocyanidol) et les ions cuivriques ne présentent pas d'activité antifongique sur Candida albicans.

Les inventeurs ont maintenant trouvé que de façon surprenante, il était possible d'obtenir un effet synergique sur l'activité antifongique d'un cation divalent et en particulier de l'ion cuivre divalent (ion cuivrique) lorsqu'on lui adjoint des OPC.

C'est pourquoi la présente invention a pour objet une nouvelle composition, notamment antifongique caractérisée en ce qu'elle renferme des oligomères procyanidoliques (OPC) et un cation divalent.

Avantageusement, le cation divalent sera l'ion cuivre divalent.

Les OPC peuvent être présents dans la composition sous forme d'un extrait végétal contenant des OPC ou sous une forme purifiée à des degrés divers, obtenue à partir d'un tel extrait végétal.

Tous les oligomères procyanidoliques actuellement connus peuvent être utilisés dans le cadre de la présente invention. La nomenclature de ces composés est fondée sur le nom de l'anthocyanidol qui est formé lorsque le polymère est traité à chaud par un acide. On peut citer par exemple le procyanidol, le prodelphinidol, ou encore le propelargonidol. Comme déjà indiqué, l'élément structural de base de ces polymères est un flavane-3 ol tel que par exemple le cathécol et l'épicathécol (constitutifs des procyanidols), le galocathécol et l'épigalocathécol (constitutifs des prodelphinidols) ou encore l'afzélechol et l'épiafzélechol (constitutifs des propelargonidols).

De façon avantageuse, les OPC seront présents sous une forme les protégeant de l'oxydation avant leur mise en contact avec le cuivre. Cette protection peut être effectuée par tout moyen, tel que par exemple une encapsulation par des liposomes ou à l'aide de glycosphères.

Parmi la famille des OPC, on citera plus particulièrement les OPC de Quebracho ou encore les OPC de pépins de raisins encapsulés ou non.

Le cation divalent peut être présent dans la composition sous la forme de n'importe quel sel. Notamment tous les sels connus et utilisables sur la peau et les muqueuses humaines, tels que les chlorures, les gluconates, les glycinates, les sulfates peuvent être utilisés. A titre d'exemple non limitatif, on peut citer le chlorure cuivrique ou le sulfate cuivrique. On utilisera de préférence le sulfate cuivrique CuSO₄.

Avantageusement, les OPC et le cation divalent sont présents dans la composition en quantités telles que la composition a une activité antifongique.

On choisira de préférence les quantités respectives d'OPC et d'ion cuivre divalent dans la composition de telle façon qu'elles permettent d'obtenir un effet synergique sur l'activité antifongique du cation divalent. En effet, et c'est là que réside l'originalité de l'invention, l'addition d'OPC à une composition comprenant des cations divalents, notamment des ions cuivre, permet d'accroître de façon notable l'activité antifongique du cation par rapport à l'activité antifongique qu'il exerce seul.

Dans le cadre de l'invention, on entend par activité antifongique, l'effet de la composition contre levure, ou un champignon donné. Elle est mesurée en ajoutant une quantité déterminée de germes de ladite levure ou champignon à une composition selon l'invention sous la forme d'une crème ou d'une solution et en dénombrant à des instants donnés après cet ajout le nombre de germes survivants par g ou ml de la composition selon qu'il s'agit, dans le modèle de mesure retenu, d'une crème ou d'une solution.

De préférence, les proportions respectives d'OPC et de cation divalent dans la composition seront de 0,01 à 1% environ en poids de cation divalent et de 0,005 % à 1% environ en poids d'OPC (rapporté en poids d'OPC purs), exprimées par rapport au poids total de la composition.

Avantageusement, selon un mode de réalisation préféré, la composition comprend :
CuSO₄ 0,5 % (exprimés en poids par rapport au poids OPC 0,02 % total de la composition).

L'invention concerne également l'utilisation d'une composition telle que définie ci-dessus en tant qu'agent antifongique, notamment en tant qu'agent antifongique sur Candida albicans.

Le Candida albicans apparaît de manière opportuniste et a tendance à se développer, en présence d'humidité, dans les conditions d'exclusivité générées par le port des couches. Il cause des dermatites fessières fortement erythémateuses en particulier dans les plis de l'aine et la région périanale.

Les compositions telles que définies sont avantageusement utilisées en tant que produit cosmétique ou pharmaceutique, notamment dermatologique, plus particulièrement pour la prévention ou le traitement des érythèmes fessiers, ou des candidoses.

L'invention a également pour objet une composition cosmétique ou pharmaceutique notamment dermatologique, plus particulièrement pour le traitement et la prévention des érythèmes fessiers, qui renferme une composition selon l'invention.

Dans les compositions cosmétiques ou pharmaceutiques selon l'invention, les compositions renfermant des OPC et un cation divalent sont présentes préférentiellement à titre d'agent antifongique. Outre le traitement et la prévention des érythèmes fessiers, ces compositions cosmétiques ou pharmaceutiques peuvent être utilisées pour le traitement et la prévention de candidoses de toutes sortes telles que par exemples les candidoses vaginales ou vulvaires (elles sont alors formulées comme produits d'hygiène intime) ou les candidoses péri buccales (perlèche). Elles peuvent également être utilisées pour la prévention et le traitement de l'intertrigo interorteil, certaines atteintes des ongles telles que l'onchomycose, ou des atteintes d'autres plis (mammaires, inguinaux) ou encore pour lutter contre les effets du diabète sur la peau.

La composition cosmétique selon l'invention peut se présenter sous toutes les formes utilisées en cosmétique, plus particulièrement de manière à pouvoir être administrée par voie locale : solution, émulsion (huile dans l'eau ou eau dans l'huile), crème, pommade, poudre, lait, lotion, gel ou pâte à l'eau conditionné selon le cas en pots ou en tubes, en flacons de verre ou de plastique et/ou éventuellement en flacons doseurs ou encore en ampoules. Les formes cosmétiques sont préparées selon les méthodes usuelles. Pour chaque forme on a recours à des excipients appropriés qui doivent avoir toutes les qualités habituellement requises. En particulier, dans le cas d'une administration locale, il doit être doué d'une grande affinité pour la peau, être parfaitement bien toléré, stable, présenter une consistance adéquate permettant une utilisation facile et agréable.

Elle peut également être formulée pour une administration orale (bain de bouche).

Elle peut également être formulée comme produit d'hygiène corporelle, notamment d'hygiène intime. On peut citer de façon non limitative les produits anti-transpirants, déodorants sous forme de spray ou gel, les shampoings, notamment antipelliculaires, les savons, les produits d'hygiène dentaire etc.. On peut envisager également d'utiliser les compositions selon l'invention dans le traitement des peaux à tendance grasse (peaux acnéiques).

En vue d'une application pour le traitement et la prévention de l'érythème fessier, la composition cosmétique ou dermatologique selon l'invention sera de préférence dépourvue de conservateur ou de parfum.

L'invention concerne une méthode de traitement cosmétique du corps humain ou animal caractérisée en ce qu'elle consiste à appliquer sur le corps humain ou animal une composition cosmétique telle que définie ci-dessus.

Enfin, l'invention a pour objet un complément alimentaire qui renferme une composition à base de cation divalent et d'OPC selon l'invention.

Ce complément alimentaire peut se présenter par exemple sous la forme d'une gélule, capsule, poudre ou d'un comprimé effervescent ou non.

Il peut être utilisé notamment pour la prévention ou le traitement d'un déséquilibre fongique pouvant résulter d'une antibiothérapie.

Les exemples suivants illustrent l'invention sans toutefois la limiter. Ils sont accompagnés des Figures 1 à 3 qui mettent en évidence l'activité antifongique d'une association OPC-CuSO₄.

La Figure 1 met en évidence l'activité antifongique synergique des OPC de Québracho associés à CuSO₄. En abcisse est reporté le temps en heures, et en ordonnée le nombre de germes de la levure Candica albicans exprimé en logarithme.
+ OPC Québracho 0.02% CuSO₄ 0.5%
O OPC Québracho 0.02%
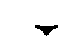 CuSO₄ 0.5%

La Figure 2 met en évidence le même type de synergie avec 0,25 % d'OPC de Québracho.

La Figure 3 est une figure analogue à la Figure 2 sauf qu'elle a été réalisée avec des OPC de pépins de raisins.
+ OPC de pépins de raisins 0.25%
O OPC de pépins de raisins 0.25% CuSO₄ 0.5%
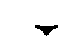 CuSO₄ 0.5%

Pour l'ensemble des exemples qui vont suivre, on décrira ci-après la méthode retenue pour mesurer l'activité levuricide de la composition testée.

Pour mettre en évidence l'activité levuricide, le produit à contaminer (100 g de crème ou 50 ml de solution selon le cas) est réparti en flacons. La contamination est réalisée à l'aide d'1 ml d'une suspension de germes faite à partir d'une culture fraîche contenant entre 10⁷ et 10⁸ germes/ml. On effectue un dénombrement du nombre de germes pour chaque suspension de germes. On quantifie ensuite, le nombre de germes revivifiables restant dans le produit contaminé à des temps compris entre t = 0 heure et t = 5 heures puis à t = 24 heures. Pour cela, à chaque temps t, on prélève stérilement 5g ou 5ml de produit qui sont ensuite dispersés dans un flacon contenant 45 ml d'un diluant neutralisant approprié au produit étudié (par exemple, tween®, lecithine ou eau peptonée). Ce flacon correspond à la dilution 10⁻¹ du produit à partir de laquelle on effectue deux autres dilutions 10⁻² et 10⁻³. Pour chacune de ces dilutions, on laisse revivifier pendant 30 minutes. On ensemence ensuite en gélose T.C.S. au malt par 1ml de dilution. On procède alors aux numérations suivantes :
- numération des solutions-mères :
   les germes présents sont dénombrés dans chaque suspension bactérienne ayant servi aux contaminations ;
- détermination du nombre de germes revivifiables en fonction du temps :
   pour chaque temps t, on note le nombre de colonies correspondant à chacune des dilutions.

A partir de ces résultats on calcule le nombre moyen de germes revivifiables par ml ou par g de produit, au temps t.
- critères d'acceptabilité du produit :
un produit sera considéré comme efficace contre le germe considéré quel que soit le germe, si au plus tard à t = 1 semaine, le nombre de germes revivifiables est inférieur à 10 germes / ml.

Le tableau 1 suivant récapitule les différentes compositions qui ont été testées sur Candida albicans (activité antifongique).

**TABLEAU 1**

| Exemple | Composition | Activité testée sur |
|---|---|---|
| 1 | CuSO₄ 0.5% | Candida albicans |
| 2 | CuSO₄ 0.5%, OPC de pépins de raisins 0.25% | Candida albicans |
| 3 | OPC de pépins de raisins 0.25% | Candida albicans |
| 4 | OPC de Québracho 0.25% | Candida albicans |
| 5 | CuSO₄ 0.5% OPC de Québracho 0.25% | Candida albicans |
| 6 | OPC de Québracho 0.02 % | Candida albicans |
| 7 | CuSO₄ 0.5 % OPC de Québracho 0.02 % | Candida albicans |

Dans les exemples, les O?C de Quebracho qui ont été utilisés sont ceux commercialisés sous la marque Radicalys® par la société GREENTECH. Il s'agit d'une forme encapsulée qui contient 2 % d'OPC purs (1 % de Radicalys contient 0,02 % d'OPC purs). Dans les exemples d'émulsion, on a utilisé la forme encapsulée par des glycosphères et dans l'exemple de pâte à l'eau, on a utilisé la forme encapsulée dans des liposomes. Les OPC de pépins de raisins sont ceux commercialisés sous la marque Berkem® par la société ADF Chimie.

Les germes contaminants sont obtenus d'une culture fraiche, soit d'une souche de Candida albicans ATCC 2091.

Le Tableau 2 indique pour chacun des exemples 1 à 7 l'évolution de la population de germes exprimée en germes/ml au cours du temps et on indique à chaque fois la concentration en germes (exprimée en germes/ml) de la solution mère de départ.

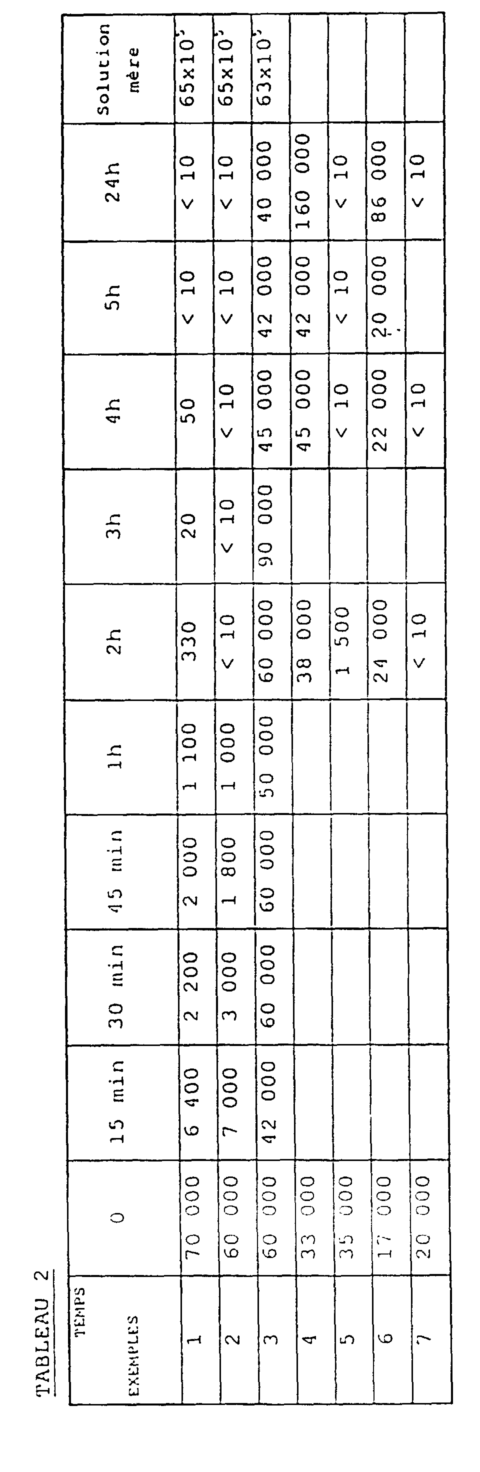

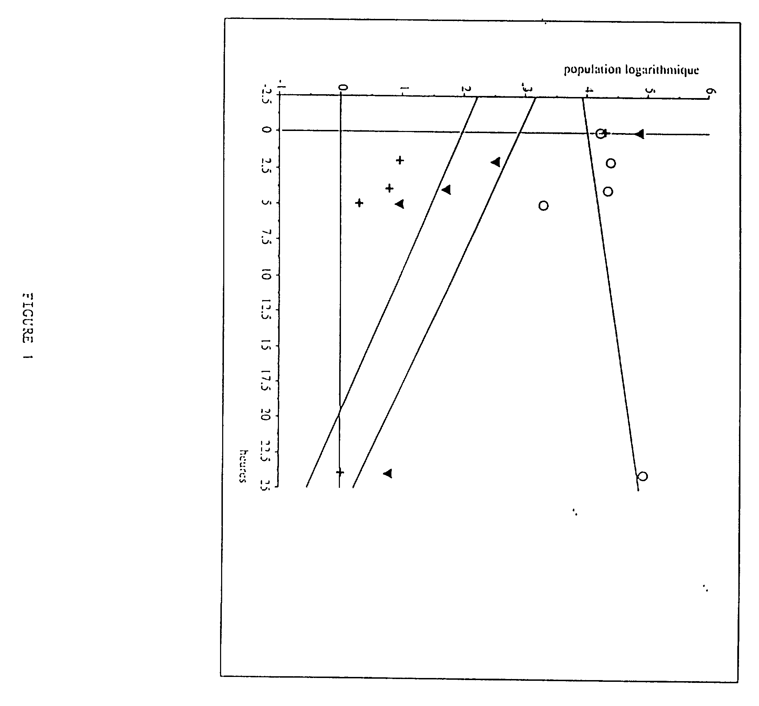

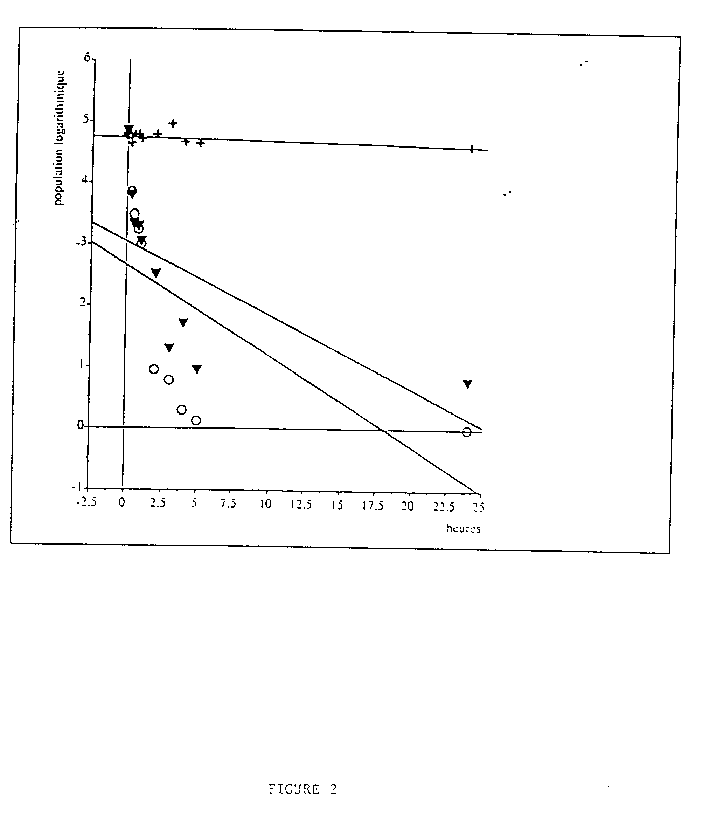

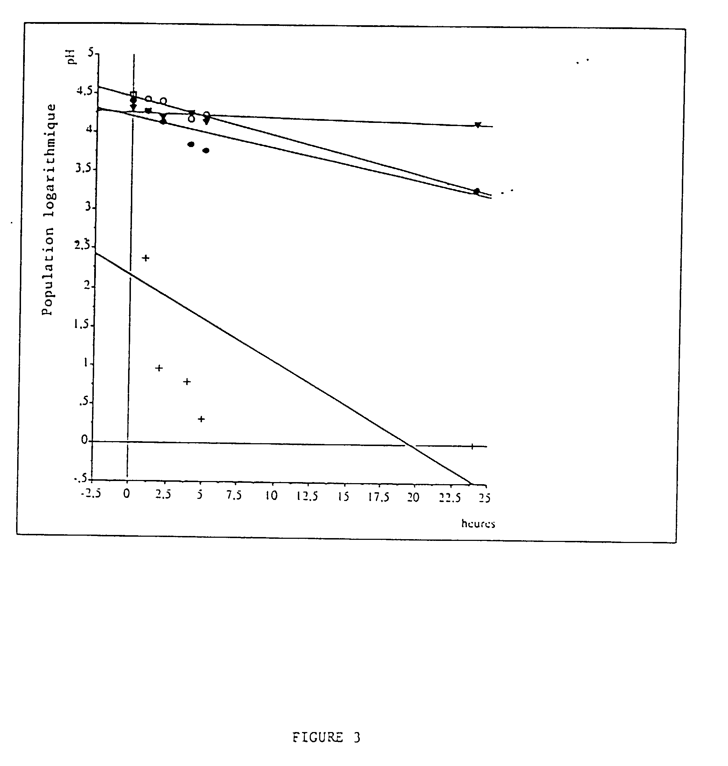

Le Tableau 2 ci-dessus en relation avec les Figures 1 à 3 permet de constater que lorsque CuSO₄ 0.5% est utilisé seul sur Candida albicans (Exemple 1), on n'observe une activité efficace qu'après 5 heures. Lorsque des OPC de pépins de raisins 0.25% sont utilisés seuls (Exemple 3), ces O?C sont inactifs. Par contre l'association des deux constituants (Exemple 2) permet d'obtenir une activité efficace après 2 heures.

Lorsque l'on considère cette fois, les O?C de Quebracho 0.25% seuls, ils ne sont pas non plus efficaces sur Candida albicans (Exemple 4). Par contre, en association avec CuSO₄ 0.5% (Exemple 5), on observe une activité efficace au bout de 4 heures.

En ce qui concerne les OPC de Québracho 0,02 %, ils sont inefficaces seuls (Exemple 6) mais efficaces ou bout de 2 heures en présence de CuSO₄ (Exemple 7).

On peut aussi conclure de ces résultats que l'efficacité de sulfate de cuivre en tant qu'antifongique est potentialisée par la présence d'OPC dans la composition.

### EXEMPLE 8 : Composition cosmétique contenant CuSO₄ et des OPC de Québracho (émulsion huile dans l'eau) :

| | % |
|---|---|
| stéarate de glycérol | 10 |
| octanoate de cétéacyl | 3 |
| végétaline | 3 |
| huile de tournesol | 4 |
| huile de germes de blé | 0,3 |
| glycérine | 5 |
| eau | qs 100 |
| cyclométhicone | 3 |
| cacétate de DL-tocophérol | 0,2 |
| conservateur | 0,2 |
| OPC de Quebracho encapsulés | 1 |
| CuSO₄ | 0,5 |

### EXEMPLE 9 : Composition cosmétique contenant CuSO₄ et des OPC de Québracho (émulsion eau dans l'huile) :

| | % |
|---|---|
| PHASE GRASSE | |
| beurre de karité | 1 |
| cire d'abeille | 2 |
| Arlacel 1690@ (surfactants commercialisés par ICI) | 4 |
| Controx KS (antioxydant) | 0,15 |
| octyl palmitate | 7 |
| huile de vaseline fluide | 10 |
| bentone MIO (épaississant) | 1 |
| oxyde de zinc | 25 |
| | |
| PHASE AQUEUSE | |
| eau | qs 100 |
| glycérine | 5 |
| sulfate de magnésium | 0,7 |
| sulfate de zinc | 0,2 |
| CuSO₄ | 0,5 |
| OPC de Quebracho encapsulés | 1 |

### EXEMPLE 10 : Composition cosmétique contenant CuSO, et des OPC de Québracho (pâte à l'eau) :

| | % |
|---|---|
| oxyde de Zinc | 20,0 |
| talc | 10,0 |
| carbonate de calcium | 10,0 |
| eau purifiée | qsp 100 |
| glycérine | 30,0 |
| hydroxyethyl cellulose | 0,2 |
| cuivre pidolate | 0,5 |
| zinc pidolate | 0,5 |
| OPC de Quebacho encapsulés | 1,0 |
| extrait glycolique de la ratanhia | 1,0 |
| hydroxypropyl guar | 0,1 |
| CuSO₄ | 0,5 |

### Exemple 11 : Composition cosmétique contenant l'ion cuivre divalent et des OPC de Québracho encapsulés (pâte à l'eau) %

| | % |
|---|---|
| | |
| - oxyde de zinc | 20,0 |
| - talc | 23,0 |
| - polyethylène glycol | 15 |
| - montmorillonite | 1,1 |
| - acide lactique | 2,0 |
| - acide tartrique | 0,1 |
| - eau purifiée | qsp 100 |
| - glycérine | 15,0 |
| - hydroxyéthycellulose | 0,7 |
| - cuivre pidolate | 0,5 |
| - zinc pidolate | 0,5 |
| - OPC de Québracho encapsulés (correspond à 0, 02% d'OPC non encapsulés) | 1,0 |
| - extrait glycolique de la Ratanhia | 1,0 |
| - hydroxypropyl guar (pas de conservateur) | 0,05 |

### Exemple 12 : Compostion cosmétique contenant l'ion cuivre divalent et des OPC de Québracho non encapsulés(pâte à l'eau) %

| | % |
|---|---|
| | |
| - oxyde de zinc | 20,0 |
| - talc | 23,0 |
| - polyethylène glycol | 15 |
| - montmorillonite | 1,1 |
| - acide lactique | 2,0 |
| - acide tartrique | 0,1 |
| - eau purifiée | qsp 100 |
| - glycérine | 15,0 |
| - hydroxyéthycellulose | 0,7 |
| - cuivre pidolate | 0,5 |
| - zinc pidolate | 0,5 |
| - OPC de Québracho non encapsulés | 0,02 |
| - extrait glycolique de la Ratanhia | 1,0 |
| - hydroxypropyl guar | 0,05 |
| - octoxyglycérine (conservateur) | 0,5 |

Les OPC de Québracho non encapsulés sont introduits dans la composition juste avant son utilisation pour le test d'activité décrit ci-après.

### Exemple 13 : Compostion cosmétique contenant l'ion cuivre divalent et des OPC de Québracho encapsulés (pâte à l'eau)

| | % |
|---|---|
| - oxyde de zinc | 20,0 |
| - talc | 23,0 |
| - polyethylène glycol | 15 |
| - montmorillonite | 1,1 |
| - acide lactique | 2,0 |
| - acide tartrique | 0,1 |
| - eau purifiée | qsp 100 |
| - glycérine | 15,0 |
| - hydroxyéthycellulose | 0,7 |
| - cuivre pidolate | 0,5 |
| - zinc pidolate | 0,5 |
| - OPC de Québracho encapsulés (correspond à 0,02 % d'OPC non encapsulés) | 1,0 |
| - extrait glycolique de la Ratanhia | 1,0 |
| - hydroxypropyl guar | 0,05 |
| - octoxyglycérine (conservateur) | 0,5 |

L'activité antifongique des trois compositions des exemples 11 à 13 est testée sur Candida albicans (souche ATCC 2091). La mesure de l'activité est réalisée selon les recommandations de la Pharmacopée française Chapitre III Janvier 1990.

Les compositions des exemples 11 à 13 sont testées pures, diluées au 1/2 et diluées au 1/10 ème. Les compositions sont artificiellement contaminées par un inoculum de micro-organismes (souche Candica albicans) compris entre 1x10⁸ et 3 x 10⁸.

Le dénombrement est effectué par dilution-neutralisation afin de connaître le nombre de germes introduit dans la composition lors de l'essai, l'activité antifongique mesurée pour des temps de contact de 5 minutes et de 30 minutes ±10 secondes) à 32°C(±1 °C).

On prépare une suspension de la souche de Candida albicans, et on effectue des dilutions en tryptone sel jusqu'à 10⁻⁶, 1 millilitre de la souche à la dilution 10⁻⁶ étant ensuite ensemencé en profondeur.

Les compositions testées sont les suivantes :
- compositions testées pures : 1 millilitre de souche (10⁸ / ml théorique) + 9 grammes de composition à tester ; concentration réelle testée : 90%
- compositions testées au 1/2 : 1 millilitre de souche (10⁸ / ml théorique) + 4 millitres de diluant R (solution tampon peptonnée au chlorure de sodium Ph7) + 5 grammes de composition à tester ; concentration réelle testée : 50 %
- composition testées au 1/10 ème : 1 millitre de souche (10⁸ /ml théorique) + 8 millilitres de diluant R + 1 gramme de composition à tester; concentration réelle testée 10%.

Un contact préalable de 10 minutes au bain-marie à 32°C (±1°C) entre la composition testée et le diluant neutralisant, est effectué afin d'obtenir l'équilibre thermique avant introduction de la souche.

Après des temps de contacts respectifs de 5 minutes et 30 minutes (±10 secondes) à 32°C (±1°C) avec la souche, 1 millilitre de la solution inoculée est prélevé et introduit dans un tube contenant 4,5 millilitres de diluant-neutralisant et 4,5 millilitres d'eau distillée stérile, et maintenue à 32°C (±1 °C) pendant 10 minutes.

Ensuite, 1 millilitre de ce mélange est ensemencé en profondeur, en milieu gélosé B (voir composition de ce milieu ci-après).

Les boîtes sont incubées 48 heures à 37 °C(± 1 °C).

On réalise un contrôle de stérilité et de fertilité des milieux utilisés. Ensuite, on effectue un dénombrement de la souche respectivement en l'absence et en présence de la composition testée.

Les compositions des milieux de culture et diluant utilisés dans le test sont indiquées ci-après.

| Diluant simple (Tryptone sel) : MERCK 12535, MERCK 15421 et/ou AES 111499 | |
|---|---|
| Peptone de caséine trypsique | 1 g |
| Chlorure de sodium | 8, 6 g |
| Eau déminéralisée qsp | 1000 ml |

| « MILIEU GELOSE B » : | |
|---|---|
| Agar aux peptones de caséine et de farine de soja : MERCK 5458 | |
| Peptone de caséine | 15 g |
| Peptone de farine de soja | 5 g |
| Chlorure de sodium | 5 g |
| Agar-agar | 15 g |
| Eau déminéralisée qsp | 1000 ml |

| « DILUANT R » : | |
|---|---|
| Solution Tampon peptonée au chlorure de sodium pH7 : MERCK 10582 | |
| Peptone de viande ou de caséine | 1 g |
| Phosphate monopotassique | 3, 56 g |
| Phosphate disodique dihydraté | 7, 23 g |
| Chlorure de sodium | 4, 30 g |
| Eau déminéralisée qsp | 1000 ml |

Le tableau 3 ci-après indique pour chacun des exemples 11 à 13 aux différentes concentrations testées, l'évolution de la population de germes au cours du temps, à savoir la réduction du nombre de cellules viables à la concentration d'essai, exprimée en ufc / ml et en logarithme.

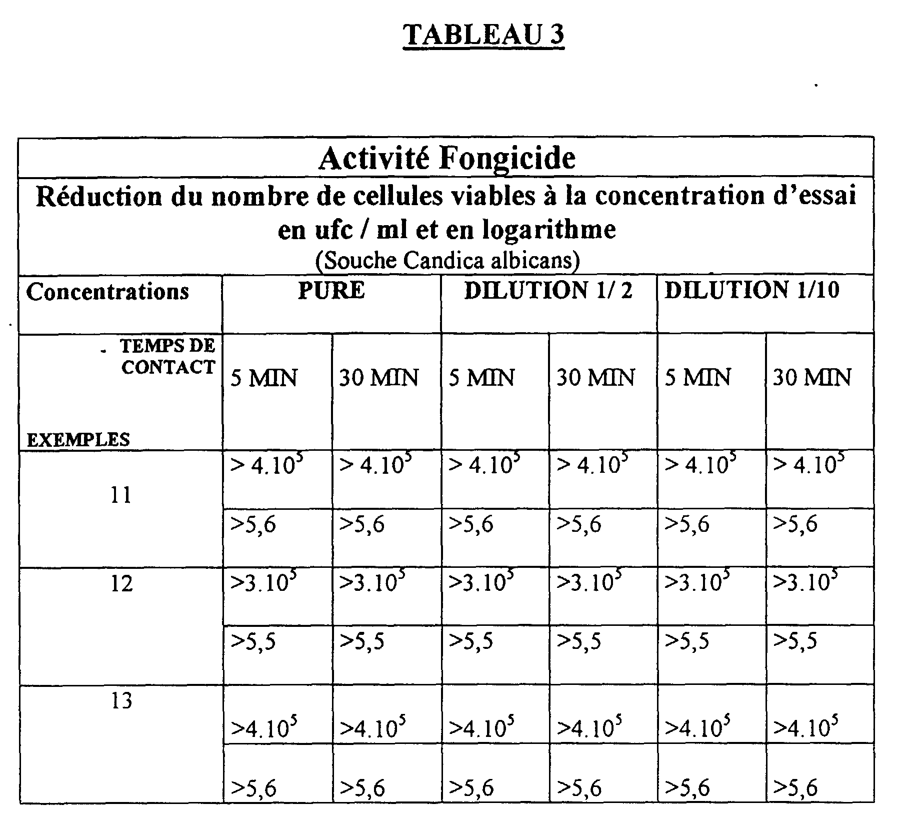

Il résulte de ces tests d'activité que les compositions de pâtes à l'eau des exemples 11 à 13 présentent chacune pure, diluée au demi ou diluée au dixième, pour Candida albicans une activité en accord avec les recommandations de la Pharmacopée française avec une réduction logarithmique à chaque fois supérieure à 5 log pour tous les temps.

## Revendications

1. Composition, notamment antifongique caractérisée en ce qu'elle renferme des oligomères procyanidoliques (OPC) et un cation divalent.

2. Composition selon la revendication 1, caractérisée en ce que ledit cation divalent est un ion cuivre divalent.

3. Composition selon l'une des revendications 1 ou 2, caractérisée en ce que lesdits OPC sont protégés contre l'oxydation.

4. Composition selon la revendication 3, caractérisée en ce que lesdits OPC sont protégés par encapsulation dans des liposomes ou à l'aide de glycosphère.

5. Composition selon l'une des revendications 1 à 4, caractérisée en ce que lesdits OPC sont choisis parmi les OPC de Quebracho et les OPC de pépins de raisins, encapsulés ou non.

6. Composition selon l'une des revendications 2 à 5, caractérisée en ce que l'ion cuivre divalent est sous la forme de sulfate cuivrique.

7. Composition selon l'une des revendications 1 à 6, caractérisée en ce que les OPC et le cation divalent sont présents en quantités telles que ladite composition a une activité antifongique.

8. Composition selon la revendication 7, caractérisée en ce les OPC et le cation divalent sont présents en quantités telles qu'on obtient un effet synergique sur l'activité antifongique du cation divalent.

9. Composition selon la revendication 7 ou 8, caractérisée en ce que le cation divalent est présent à raison de 0.01 % à 1% environ en poids, par rapport au poids total de la composition.

10. Composition selon l'une quelconque des revendications 7 à 9, caractérisée en ce que les OPC sont présents à raison de 0.005 % à 1% environ en poids d'OPC, par rapport au poids total de la composition.

11. Composition selon l'une des revendications 9 à 11, caractérisée en ce qu'elle comprend du CuSO₄ à une concentration de 0,5 % et des OPC à une concentration de 0,02 % exprimées en poids de la composition.

12. Utilisation d'une composition selon l'une quelconque des revendications 1 à 11 en tant qu'agent antifongique.

13. Utilisation d'une composition selon l'une quelconque des revendications 1 à 11 en tant qu'agent antifongique sur Candida Albicans.

14. Utilisation d'une composition selon l'une des revendications 1 à 11 en tant que produit cosmétique, notamment pour la prévention ou le traitement des érythèmes fessiers.

15. Composition selon l'une des revendications 1 à 11, à titre de produit pharmaceutique, notamment dermatologique.

16. Composition selon l'une des revendications 1 à 11, à titre de produit dermatologique pour la prévention ou le traitement des érythèmes fessiers.

17. Composition selon l'une des revendications 1 à 11 à titre de produit dermatologique pour la prévention et le traitement des candidoses notamment vaginales, vulvaires, péri buccales.

18. Composition cosmétique ou pharmaceutique, notamment dermatologique, plus particulièrement pour la prévention ou le traitement des érythèmes fessiers ou des candidoses, caractérisée en ce qu'elle renferme une composition selon l'une quelconque des revendications 1 à 11.

19. Composition selon la revendication 18, caractérisée en ce qu'elle se présente sous forme d'une solution, émulsion, crème, pommade, poudre, lait, lotion, gel ou pâte à l 'eau.

20. Composition selon la revendication 18 ou 19, caractérisée en ce qu'elle se présente sous forme d'un produit d'hygiène corporelle, notamment hygiène intime.

21. Méthode de traitement cosmétique du corps humain ou animal, notamment pour le traitement et la prévention des érythèmes fessiers caractérisée en qu'elle consiste à appliquer sur le corps humain ou animal une composition cosmétique selon l'une des revendications 18 à 20.

22. Complément alimentaire caractérisé en ce qu'il renferme une composition selon l'une des revendications 1 à 11.

23. Complément alimentaire selon la revendication 22 caractérisé en ce qu'il se présente sous la forme d'une gélule, capsule, d'une poudre, ou d'un comprimé effervescent ou non.
